# EUROPEAN PATENT SPECIFICATION

(11) **EP 1 137 344 B1**
(45) Date of publication and mention of the grant of the patent: **17.08.2005**
(21) Application number: 99963801.8
(22) Date of filing: 03.12.1999
(51) Int. Cl.: A23L 1/29, A23D 7/015, A61K 9/127

(54) **PREPARATION OF CHOLESTEROL REDUCING EDIBLE PRODUCTS BY MIXING PLANT STEROLS/STANOLS IN A MELT OF A FOOD EMULSIFIER**
HERSTELLUNG CHOLESTEROLREDUZIERTER ESSBARER PRODUKTE DURCH MISCHEN VON PFLANZENSTEROLEN/STANOLEN IN EINER SCHMELZE EINES LEBENSMITTELEMULGATORS
PREPARATION DE PRODUITS ALIMENTAIRES REDUISANT LE CHOLESTEROL PAR MELANGEAGE DE STEROLS/STANOLS D'ORIGINE VEGETALE DANS UN EMULSIFIANT ALIMENTAIRE FONDU

(30) Priority: 08.12.1998 SE 9804253
(43) Date of publication of application: 04.10.2001
(73) Proprietor: TRIPLE CROWN AKTIEBOLAG, S-113 47 Stockholm (SE)
(72) Inventor: DAHLSTEN, Carl-Johan, S-105 46 Stockholm (SE); BURLING, Hans, S-105 46 Stockholm (SE); STRINNING, Olof, S-105 46 Stockholm (SE)
(86) International application number: PCT/SE1999/002263
(87) International publication number: WO 2000/033669

(56) References cited:
- EP-A1- 0 289 636
- EP-A1- 0 897 671
- Remington's Pharmaceutical Sciences, Fifteenth edition, 1975, Mack Publishing Company, Easton, Pennsylvania 18042, pages 1452-1456, XP002926631.

## Description

The present invention generally relates to a method of dissolving natural sterols or stanols in water based product systems, and the invention is more particularly concerned with a method of preparing an edible cholesterol reducing or inhibiting agent and a product, like a food product, a health food product or a pharmacological product containing such cholesterol reducing or inhibiting agent.

The invention also relates to a product containing an additive of such a cholesterol reducing/inhibiting agent.

Cardiovascular diseases are commonly spread among a large part of the western population. An important ground for such diseases is an increased serum cholesterol content in the blood, in particular LDL (Low-Density Lipoprotein) cholesterol (see Pollak, OJ, " Reduction of Blood Cholesterol in Man. Circulation" , 7, 702-706 [1953]).

The serum cholesterol level of blood serum can be reduced mainly in two ways, either by reducing the cholesterol absorption in the intestinal system, or by reducing the cholesterol synthesis in the liver. The cholesterol in the intestinal system leads its origin both from the food, about 0.3 g per day, and from the cholesterol which is transported to the gastrointestinal tract via the bile. The latter amount is about 3-5 times greater than the dietary supply. (See Wilson, MD & Rudel, LL. " Review s of Cholesterol Absorption with Emphasis on dietary and biliary Cholesterol", Journal of Lipid Research, 35, 943-955, [1994]).

There are to-day medicines which can control both the absorption of cholesterol in the intestinal system and the synthesis of cholesterol in the liver. The principle for reducing the absorption builds on absorbing cholesterol to a substance having ion exchange properties. The cholesterol synthesis can be reduced by suppressing a key enzyme in said process. By drug treatment a reduction of up to 25-30% of the cholesterol content in the blood can be reached. Such therapies, however, can not be made without adverse effects and at high drug costs. Therefore there is a great national interest to reduce the LDL (Low Density Lipoprotein) content in the blood emanating from the food intake.

It has been known since long that plant sterols are capable of reducing the absorption of cholesterol in the intestinal system. The most important plant sterols are sitosterol, campesterol and sitostanol, respectively. Said compounds normally are included in the food. For instance cereals are rich sources for plant sterols. It is considered that the intake of plant sterols and plant stanols in the normal food amounts to about 0.3-0.5 g per day.

Chemically cholesterol, sitosterol, sitostanol and campesterol are closely related. What differs is only the substitution of the 24th carbon atom in the carbon skeleton. In cholesterol there is a lack of a substituent, whereas campesterol has a methyl group bound therein and sitosterol has an ethyl group. Stanols have a double bond less in the carbon skeleton than the corresponding sterol. The physical-chemical properties are almost identical except for the possibility of transportation through the intestinal wall. The uptake of cholesterol from the intestinal tract is, on average, about 50%, with a great individual variation, campesterol about 10% and sitosterol about 5%. A little amount of the plant sterols are in the nature hydrated. The hydrated variants, that is the stanols, are completely incapable of moving through the intestinal wall and into the blood circulation system.

It is known that the existence of plant sterols and plant stanols in active state can lead to a reduction of the absorption of cholesterol in the blood by between 50 and 70%, and this means that the normal absorption of cholesterol is reduced from 50% and down to 15-25%. Said reduced absorption, in turn, leads to a reduction of the LDL (Low-Density Lipoprotein) content in the blood by about 8-12%.

It is believed that the plant sterols/stanols substitute cholesterol in the micelles, which is a necessary physical form for the cholesterol to be absorbed in the intestinal tract. The exact appearance of said micelle structures is not yet made clear, but in such particles mono/diglycerides, bile salts, triglycerides and water are present in a liposome like structure. It is also known that esterified plant sterols/stanols, like cholesterol, which are received via the food, are hydrolysed in the intestinal tract by the lipoenzyme of the intestinal tract. The active form consequently is the free sterol/stanol in a micellary liposome structure.

It is obviously interesting to enrich food-stuffs with plant sterols or plant stanols. The problem is the availability of sterols and stanols and the solubility thereof in the intestinal liquid, since it is known that it is difficult to solve the natural sterols or stanols both in oil (about 1 %) and in water (less than 1 %).

In a known case this problem has been solved by esterifying the plant sterols. By this method the sterols become fat soluble to about 20% and can be added to a fat mixture, for instance to margarine. Clinical investigations have proved that, for reducing the cholesterol content by 8-12%, there is a need of intake of 2 g calculated as plant sterol. Corresponding experiences have been observed as concerns plant stanols. EP 0 289 636 discloses emulsified sterol compositions.

In a research report information was recently presented that corresponding plant sterol esters, but without an intermediate hydrogenation, have the same cholesterol reducing effect as the above mentioned hydrogenated variant. The said method also has been practised for enriching salad oil. When admixing of plant sterols or plant stanols in crystallinic form there is a poor availability. There is need for an over-dosing by a factor 5-10, or even 10-20 g, for obtaining the same effect for the above mentioned esterifying process. The disadvantages of said esterifying process is the chemical modification of the plant sterol/stanol molecule. Both hydrogenation and esterification are processes which are unnatural in food handling. This involves both use of metallic catalysts (Ni) and of solvents. For making sure that no harmful substances are added to the food-stuff it is necessary to perform an extensive purification. Without hydrogenation the process is somewhat more simple, but still the fact remains that the molecule is chemically modified in an unnatural way. The process costs also are important. The border against medicines is unclear for the above mentioned variants of adding active forms of plant sterols.

In the publication Heinemann, T, G-A et al "Mechanisms of Action of Plant Sterols on inhibition of Cholesterol Absorption, Comparison of Sitosterol and Sitostanol", Eur. Journal of Clin. Pharmacol. 40 (Supplement), p 59-63 [1991] there are described clinical tests, whereby sitosterol is dissolved in a monoolein but without dispersing the solution in a water containing medium for obtaining liposomes.

Liposomes are very small particles of lipoidalic material, which are kept emulsified in the form of a double layer or an "invisible fat". Liposomes are very small bag like particles, often having a diameter of only 25-100 nm, which can serve as carriers for various substances like vitamins, bacteriae etc. and they are favourable in that the liposome shell is dissolved at a place of the intestinal tract, whereby said carried substance is sucked up through the intestinal wall.

A basic problem according to the invention has been to provide a liposome structure, in which sitosterols or sitostanols can be introduced for supply to the human body, preferably in the small intestine, so that the sterols/stanols can contribute to a reduction of the cholesterol content in the body. The preparation of liposomes have been subject to problems, in particular since the previous preparation of liposomes have involved, among other things, use of unpolary solvents, which processes are unsuitable for handling of food stuffs.

It has shown possible to prepare liposomes in a water containing system of sitosterols/sitostanols including a suitable food emulsifier. In the present case there is used a hydrophilic emulsifier. A prerequisite therefore is, however, that the content of emulsifier is much higher than the content of sitosterol/sitostanol, for instance that the content of emulsifier is nearly twice the content of sitosterol/sitostanol.

According to the invention the above mentioned inconveniences and disadvantages involved in the previously known method are solved by a method according to which
- sterols or stanols having cholesterol reducing properties are dissolved or mixed in a melt of food emulsifiers in a relationship according to which the food emulsifier is present to a higher degree than the degree of sitosterol/sitostanol,
- said solution or mixture is mixed into a protein containing product at a temperature of 40-100°C,
- the solution or mixture is homogenized, whereby an emulsion of liposomes containing plant sterols/stanols is obtained in the protein containing product,
- and the sterol/stanol containing emulsion is thereafter admixed to food stuffs of different types, like in various types of milk products, edible fats.

By this method plant sterols/stanols are made soluble by being encapsulated in a liposome structure, analogously to what occurs naturally in the intestine. The method involves dissolving of a mixture of plant sterols/stanols in a food emulsifier, for instance an acid esterified monoglyceride. If the circumstances are the correct ones, that is that the relationship between sterol/stanol and the food emulsifier, respectively, the temperature and the homogenization relationships, there is formed a lamellary structure or a secondary layer of the monoglyceride containing plant sterols/stanols. At the specified circumstances said lamellary structures are transferred into liposomes having one or more secondary layers. The fact that this occurs has been confirmed by transmission electron microscopy study. The size of the liposomes is in this case in the interval of 0.1-2 µm which size depends on the aggregation degree.

The plant sterol/stanol molecules thus made soluble can be added to all kinds of edible products, like in milk, as an additive to products which can be curdled, like cheese, in cooking fats, in yoghurt, in acidified milk products, in powdered milk, in cream powder, in chocolate drink powder, in gruels, in health food products, in pharmacological preparations. The availability of said preparation is as good as a solution in fats of the corresponding esterified plant sterol products.

The method according to the invention leads to the great advantage that the use of the product is not restricted to fat products. On the contrary the entire market is open for enriching of food stuffs of all kinds, health food products, pharmacological preparations.

It seems that the mechanism for the reduction of cholesterol is that the product reduces the absorption of cholesterol in the intestine; said reduction increases the cholesterol synthesis in the liver but not enough for compensating the reduced supply from the intestine. The net effect thus is that the cholesterol content in the blood is reduced. Further advantages and characteristics of the invention are defined by the subclaims.

Now the invention is to be explained more in detail with reference to a number of examples of the method for preparing edible cholesterol reducing products.

### Example 1

A cholesterol reducing emulsion for use in food products was prepared in that 10 g of a lipophilic emulsifier in the form of a conventional monoglyceride (Dimodan RT® from Danisco) was melted at a temperature of about 90°C, and in that 7 g of a conventional sitosterol preparation from soy (60% declared sitosterol) was dissolved in the melt. The melt was added, under vigorous stirring, to 1 litre skim milk having a temperature of 72°C and was pumped through a dairy homogenisator (Rannie laboratory model) at a pressure drop in the homogenisator of 250 bar, whereby an emulsion was formed.

The emulsion, thus formed, was studied in microscope, and it could be established that the emulsion hardly contain any liposome like particles at all. This proves that a lipophilic emulsifier does not give a sufficient amount of liposomes.

### Example 2

For finding out the importance of the relationship between sitosterol/sitostanol and emulsifier for obtaining liposome particles, in particular an optimum part of liposomes, example 1 was repeated, whereby there was used a hydrophilic emulsifier (Citrem N12® from Danisco) with the relationship between sterol or stanol in respect to the emulsifier; a) 2, b) 1.5, c) 1, d) 0.75, e) 0.5 and f) 0.3.

The tests showed that there were hardly formed any liposomes at all when the amount of sterol or stanol was higher than the amount of emulsifier (cases a) and b) above); that there was formed a very little amount of liposomes when the relationship between sterol or stanol to emulsifier was about 1:1 (case c above); that the content of liposomes increased to an optimum when the relationship between sterol or stanol to emulsifier was 1:2, and that the content of liposomes thereafter decreased slightly at still decreasing relationship of sterol or stanol to emulsifier (cases d, e, f above), and that other problems appeared at higher content of emulsifier than at the relationship 1:2 (case e above).

It could therefore be established that it was necessary to foresee an excess of emulsifier in relation to sterol or stanol in order to obtain a noticeable amount of liposomes, and that a preferred relationship between sitosterol/sitostanol and emulsifier was 1:2.

### Example 3

The same method as in example 2 was repeated but for the difference that the sitosterol containing preparation was made from tall oil having a declared sitosterol content of 90%. The ready product differed only marginally from products according to example 2.

### Example 4

The emulsions according to examples 2 and 3 were acidified using a conventional soured milk culture (Streptococcus Lactis) over night at 18°C. The soured milk thereby formed had a normal appearance and consistency. The taste was slightly more flat than that of ordinary soured milk. When mixed with strawberry purée the product could not, however, be differed from standard strawberry soured milk.

### Example 5

The method according to example 1 was repeated but for the difference that the emulsifier (monoglyceride) used in this example was a glyceride (Dimodan M090, which is a lipophilic emulsifier) containing more monoolein, and that the temperature of the skim milk was 45°C. The milk emulsion which was formed had a rancid off-flavour, probably emanating from the monoglyceride which was used. The product also had a more unsuitably large particle size than in the product of example 1. The average particle size was 25 µm. No liposomes could be traced at microscopical examination.

### Example 6

The method according to example 1 was repeated but for the difference that a more hydrophilic emulsifier was used, Lactodan®, which is a monoglyceride which has been esterified by means of lactic acid. The relationship between emulsifier and sterol was 2:1. As a result there was obtained a product having a neutral-to-good taste and an average particle size which was as low as about 1.0 µm. At microscopic examination large amounts of liposomes were found.

### Example 7

The same method as in example 6 was repeated but for the difference that the food emulsifier which was used was based on Lactem P22® and soy lecithin (MC This AF from Lucas Meyer). The milk emulsion thereby formed had a yellowish colour tone and a strong off-flavour of soy and fish. At microscopical synthesis a fewer amount of liposomes were observed than the corresponding value of example 6. The particle size was 1-20 µm. Parallel tests showed that the emulsion could be improved by searching optimum combinations or parameters for pressure and temperature. The off-flavour of the product was considered disturbing, but it could be established that lecithin might be an excellent emulsifier, provided the taste problems could be solved.

### Example 8

The same method as in example 2 was repeated but for the difference that the pressure and the temperature was varied during the homogenization. Three different homogenization temperatures were tested, namely 60, 80 and 90°C, and two different homogenization pressures were tested, namely the pressures 60 bar and 500 bar. The results of the tests generally showed a less good product than in example 2. The best results were obtained with a homogenization temperature of 80°C and a homogenization pressure of 60 bar.

### Example 9

The same method was repeated as in example 2, but for the difference that a so called "microfluidizer" was used for the homogenization. In this case the pressure drop was of the magnitude 1.000 bar. The average particle size of the product was measured to 2 µm, but there was a wide spreading of particle size. At microscopical examination liposome particles were observed.

### Example 10

The same method was repeated as in example 3 but for the difference that 30 g monoglyceride esterified by lactic acid and 15 g of a sitosterol preparation from soy was added to milk having a fat content of 1 %. The emulsion which was formed had a good distribution of liposome particle size and an average particle size of 5 µm. This more concentrated emulsion is intended to be used for enriching of food stuffs, or for use as pharmacological preparations. The product therefore was freeze dried and was sent for forming tablets thereof or for eventual later clinical tests. After the powder was dissolved in water liposome type particles could still be seen using microscope.

### Example 11

1 kg of a conventional esterified monoglyceride (30% Citrem N12® and 70% Lactom P22®) was melted together with 0.5 kg sitosterol preparation from tall oil (90% declared sitosterol). The melt was added to 100 litre light milk (0.5% fat content), and the mixture was vigourously mixed by means of an Ultraturrax® mixer, whereupon the mixture was sterilized in an Alfa Laval UHT sterilizator at 144°C for 4 seconds, was cooled by vacuum cooling to 78°C and was sterile homogenized at a pressure drop of 200 bar. The milk emulsion thereby formed was cooled to a final temperature of 8°C. The final milk emulsion had a typically cooked taste and a slight off-flavour of tallow. It was found that the milk emulsion contained a noticeable amount of liposome particles. The distribution of particle size was examined and was found to be better than the optimum product in example 3, showing an average particle size of 1.3 µm. The milk thus prepared was used as a base material for preparing various milk based products, namely chocolate milk, sour milk, yoghurt, soft unripen cheese of quarg type and hard cheese:
a) The chocolate milk was prepared by adding 2% of cocoa powder and 8% sugar, whereupon the mixture was heated to 80°C and was thereafter cooled. The taste was quite excellent.
b) The sour milk was prepared like in example 4. The soured milk thereby formed had a good consistency but had slightly poor aroma. After strawberry concentrate had been added the product was considered to be an excellent strawberry soured milk.
c) Yoghurt was prepared using a normal yoghurt culture according to the same principle as under point b) above, except that the acidification was conducted at a temperature of 42°C for 7 hours. This resulted in an excellent product.
d) Quarg was prepared in that 2 litres of the soured milk was poured over a filter and the whey was allowed to flow off over night in a refrigerated room. In the whey which was collected there was found about 3% of the theoretical amount of sitosterol. The remaining 97% of sitosterol therefore is considered to be present in the quarg. The taste was flat, but after the product had been spiced with herb spices and pepper there was obtained a product having a good taste.
d) Hard cheese was prepared in that 30 litres of milk was curdled by adding rennet to the milk. The coagulum thereby formed which was abnormally soft, was cut to pieces, was salted and was pressed together in a press cloth. The yield of cheese was normal, and this shows that the liposome particles had been collected in the casein net structure. Liposome like particles which could be found in the cheese curd at microscopic examination.

### Example 12

A melt of Citrem LR 10/J® and sitosterol according to example 11, in the relationship of 2:1, was pressed through a membrane filter having a pore size of 5-8 µm into milk having a fat content of 5% (so called membrane emulsification). The temperature was 90°C. The resulting emulsion had a very narrow distribution of size with an average particle size of 8 µm. After homogenisation of said emulsion the distribution of size became still more narrow with a particle size of about 1 µm. This consequently is a good way of preparing an emulsion in the future having a very narrow distribution of particle size.

Further tests were made in order to investigate the possibility:
- to alternatively use sterols from soy, rape, tall or palm oil;
- to alternatively use different types of emulsifiers like monoglycerides and derivates thereof with organic acids like lactic acid and citric acid, polyglycerides, lecithin of egg, soy, rape;
- to alternatively make use of different types of homogenizing (conventional homogenization machine, membrane emulsification machine, colloid mill);
- to prepare sterol containing products like powdered milk, cream powder, gruels, health food products, different types of cheese;
- to vary the sterol content of the prepared product between 0.1 and 10% by weight.

It was shown that all tested alternatives were useful, but that the taste of the prepared product, eventual off-flavour and the average particle size could vary slightly, however only within reasonable and acceptable limits.

## Claims

1. A method of preparing cholesterol reducing edible products like food stuffs, **characterized in that**
- plant sterols or plant stanols having cholesterol reducing properties are dissolved or mixed in a melt of a food emulsifier in a relationship in which the content of food emulsifier is higher than the content of sterol/stanol,
- said solution or mixture is admixed to a protein containing product at a temperature of 45-100°C,
- the solution or mixture is homogenized, whereby an emulsion of liposome particles containing the sterols and/or stanols is formed in the protein containing product,
- and **in that** the resulting sterol/stanol containing liposome solution is mixed into food stuffs and/or is used as a health cost preparation.

2. A method according to claim 1, **characterized in that** the plant sterols or the plant stanols mainly are sitosterol, sitostanol and/or campesterol.

3. A method according to claim 1 or 2, **characterized in that** the sterol product preferably is mixed into the protein containing product at a temperature of 60-85°C.

4. A method according to claim 1, 2 or 3, **characterized in that** the sterol sources are natural sterols, preferably plant sterols for instance from soy, rape, tall and/or palm oil.

5. A method according to any of claims 1-4, **characterized in that** the emulsifier which is used is a food emulsifier like monoglyceride and derivates thereof with organic acids, polyglycerole esters, lecithin of egg, soy, rape in a relationship of emulsifier to sterol/stanol which is considerably over 1:1, preferably 2:1.

6. A method according to any of the preceding claims, **characterized in that** the protein containing product is a leguminous plant protein solution, like soy protein, milk protein solution.

7. A method according to any of claims 1 - 6, **characterized in that** the protein containing product is milk having a fat content of up to 5%.

8. A method according to any of the preceding claims, **characterized in that** the homogenization is performed by means of an equipment known per se like a homogenization machine, a membrane emulsification machine or a rotatable homogenization head of the type colloid mill.

9. A method according to any of the preceding claims, **characterized in that** the homogenization is performed at a pressure drop of 60-1000 bar, or preferably a pressure drop of 100-250 bar.

10. A method according to any of the preceding claims, **characterized in that** a sterol containing liposome solution, in a content of 0.1-10% by weight, or preferably 1-3% by weight, is mixed into a food product.

11. An edible product prepared according to any of the preceding claims, and containing the cholesterol reducing agent, **characterized in that** the edible product is a food product or a pharmacological preparation in which the cholesterol reducing agent is a liposome solution of a sterol or a stanol in combination with an emulsifier and dissolved, by homogenization, in milk to form liposomes containing the cholesterol reducing agent.

12. An edible product according to claim 11, **characterized in that** the cholesterol reducing agent is an emulsion formed by one or more sterols and/or stanols, preferably plant sterols and/or stanols from soy, rape, tall and/or palm oil which have been emulsified with a food emulsifier like monoglycerides and derivates thereof with organic acids, polyglycerole esters, lecithin of egg, soy, rape etc. in a relationship between the food emulsifier and sterol/stanol which is greater than 1:1, preferably 2:1.

13. An edible product according to claim 12 or 13, **characterized in that** the cholesterol reducing emulsion in the form of a sterol/stanol containing liposome solution, is contained, in a content of 0.1-10% by weight, or preferably 1-3% by weight of the product, as an additive in milk products, in cooking fats, in yoghurt, in acidified milk products, in powdered milk, in cream powder, in chocolate drink powder, in gruels, in health food products, in pharmacological preparations.

## Patentansprüche

1. Eine Methode zur Herstellung von cholesterinsenkenden essbaren Produkten wie Lebensmitteln, **dadurch gekennzeichnet dass**
- Pflanzensterine oder Pflanzenstanole mit cholesterinsenkeder Wirkung in einer Schmelze eines Lebensmittelemulgators in ein Verhältnis, wo der Anteil des Emulgators grösser ist als der Anteil des Sterin/Stanol, gelöst oder gemischt werden
- die benannte Lösung oder das Gemisch mit einem proteinenthaltenden Produkt bei einer Temperatur von 45-100 °C gemischt wird
- die Lösung oder das Gemisch homogenisiert wird, wobei eine Emulsion von Liposompartikeln die die Sterine/Stanole enthalten in dem proteinhaltigen Produkt gebildet wird
- und dass die resultierende Sterin/Stanol enthaltende Liposomlösung mit dem Lebensmittel gemischt wird und/oder als Heilkost verwendet wird.

2. Eine Methode wie in Anspruch 1, **dadurch gekennzeichnet dass** die Pflanzensterine oder Pflanzenstanole hauptsächlich aus Sitosterin, Sitostanol und/oder Campesterin bestehen.

3. Eine Methode wie in Anspruechen 1 oder 2, **dadurch gekennzeichnet dass** das Sterinprodukt bevorzugt mit dem proteineinhaltigen Produkt bei einer Temperatur von 60 - 85 °C gemischt wird.

4. Eine Methode wie in Anspruechen 1, 2 oder 3,**dadurch gekennzeichnet dass** die Sterinquellen natuerliche Sterine, bevorzugt Pflanzensterine z. B. von Soja-, Raps-, Tall- und/oder Palmöl sind.

5. Eine Methode wie in jene von Anspruechen 1 - 4, **dadurch gekennzeichnet dass** der verwendete Emulgator ein Lebensmittelemulgator wie Monoglyceride, und Derivate davon mit organischen Säuren, Polyglycerinen, oder Lecithin aus Eiern, Soja, Raps in einem Verhältnis von Emulgator zu Sterin/Stanol wesentlich ueber 1:1, bevorzugt 2:1 ist.

6. Eine Methode wie in jene von den vorgehenden Anspruechen, **dadurch gekennzeichnet dass** das proteinhaltige Produkt eine Lösung von leguminose Pflanzenprotein wie Sojaprotein oder Milchprotein ist.

7. Eine Methode wie in jene von den Anspruechen 1 - 6, **dadurch gekennzeichnet dass** das proteinhaltige Produkt Milch mit einen Fetthalt bis 5% ist.

8. Eine Methode wie in jene von den vorgehenden Anspruechen, **dadurch gekennzeichnet dass** die Homogenisierung in handelsueblicher Apparatur wie Homogenisierungsapparatur, Membran Emulgator Apparatur oder einem rotierenden Homogenisierungskopf vom Typus Kolloid Muehle stattfindet.

9. Eine Methode wie in jene von den vorhergehenden Ansprueche, **dadurch gekennzeichnet dass** die Homogenisierung unter einen Druckabfall von 60 - 1000 Bar oder bevorzugt einen Druckabfall von 100 - 250 Bar durchgefuert wird.

10. Eine Methode wie in jene von den vorhergehenden Anspruechen, **dadurch gekennzeichnet dass** die sterinhaltige Liposomlösung von 0,1 - 10 Gewicht % oder bevorzugt 1 - 3% in ein Lebensmittel eingemischt wird.

11. Ein essbares Produkt, wie in jene von den vorhergehenden Anspruechen hergestellt , **dadurch gekennzeichnet dass** das essbare Produkt ein Lebensmittel oder ein pharmaceutisches Präparat ist, bei dem das cholesterinsenkende Agens eine Liposomlösung von Sterine oder Stanole in Kombination mit einem Emulgator und gelöst durch Homogensierung in Milch um Liposomen zu bilden, die das cholesterinsenkende Agens enthalten.

12. Ein essbares Produkt wie in jene von den vorhergehenden Anspruechen hergestellt, **dadurch gekennzeichnet dass** das cholesterinsenkende Agens eine Emulsion von einem oder mehreren Sterinen und/oder Stanolen, bevorzugt Pflanzensterinen aus Soja-, Raps-, Tall- und/oder Palmöl, die mit einem Lebensmittelemulgator wie Monoglyceriden und Derivaten davon mit organischen Säuren, Polyglycerol Estern, Lecithin aus Eiern, Soja oder Raps emulgiert in einem Verhältnis zwischen Emulgator und Sterin/Stanol grösser als 1:1, bevorzugt 2:1 sind.

13. Ein essbares Produkt wie in jene von den vorhergehenden Anspruechen hergestellt, **dadurch gekennzeichnet dass** die cholesterinsenkende Emulsion in Form von einer Sterin/Stanol enthaltenden Liposomlösung mit einem Gehalt von 0,1 - 10 Gewichts % oder bevorzugt 1 - 3 Gewichts % von dem Produkt, als ein Zusats zu Milchprodukten, in Kochfett, in Yoghurt, in sauren Milchprodukten, in Trockenmilch, in Trockensahne, in Trinkchocolade Pulver, in Mehlsuppe, in Gesundheitskost Produkten, in pharmaceutischen Preparationen vorhanden ist.

## Revendications

1. Un procédé de préparation de produits diminuant le taux cholestérol comme des substances alimentaires **caractérisé en ce que**
- des stérols végétaux ou des stanols végétaux, ayant des propriétés réductrices de cholestérol, sont dissous ou mélangés dans un fondant d'agent émulsifiant alimentaire dans un rapport tel que le contenu en agent émulsifiant alimentaire est plus élevé que le contenu de stérol/stanol
- ladite solution ou ledit mélange est ajouté à un produit contenant une protéine à une température comprise entre 45 et 100°C,
- la solution ou le mélange est homogénéisé, après quoi une émulsion de particules liposomiques contenant les stérols et/ou stanols se forme dans le produit contenant la protéine,
- et cette solution liposomique contenant stérol/stanol est mélangée à des produits alimentaires et/ou est utilisée dans une préparation d'aliments naturels.

2. Un procédé selon la revendication 1, **caractérisé en ce que** les stérols végétaux ou les stanols végétaux sont principalement des sistosterols, des sitostanols et/ou des campesterols.

3. Un procédé selon la revendication 1 ou 2, **caractérisé en ce que** le composé stérol est préférablement mélangé au produit contenant la protéine à une température comprise entre 60 et 85°C.

4. Un procédé selon la revendication 1, 2 ou 3, **caractérisé en ce que** les stérols sont de sources naturelles, préférablement des stérols végétaux par exemple à partir du soja, de colza et de l'huile de pin.

5. Un procédé selon la revendication 1-4, **caractérisé en ce que** l'agent émulsifiant utilisé est un agent émulsifiant alimentaire comme un monoglycéride et des dérivés de celui-ci avec des acides organiques, des esters de polyglycérol ou/et de la lécithine d'oeuf, de soja, de colza avec un rapport entre agent émulsifiant et stérol/stanol largement supérieur à 1:1, preférablement 2:1.

6. Un procédé selon les revendications précédentes, **caractérisé en ce que** le produit contenant la protéine est une solution de protéine végétale légumineuse comme la protéine de soja, de lait et/ou d'autres sources de protéines.

7. Un procédé selon quelles que soient les revendications 1-6, **caractérisé en ce que** le produit contenant la protéine est du lait ayant une teneur en graisse jusqu'à 5%.

8. Un procédé selon les revendications précédentes, **caractérisé en ce que** l'homogénéisation est assurée à l'aide d'un équipement connu essentiellement comme une machine d'homogénéisation, une machine d'émulsification de membrane ou une tête rotative d'homogénéisation du type moulin de colloïde.

9. Un procédé selon les revendications précédentes, **caractérisé en ce que** l'homogéneisation est réalisée à une baisse de pression de 60-1000 bars ou préférablement à une baisse de pression de 100-250 bars.

10. Un procédé selon les revendications précédentes, **caractérisé en ce que** la solution liposomique contenant le stérol, à une concentration de 0.1-10% en poids ou préférablement 1-3% en poids est mélangée à un produit alimentaire.

11. Un produit comestible préparé selon les revendications précédentes, et contenant l'agent réducteur de cholestérol, **caractérisé en ce que** le produit comestible est un produit alimentaire ou une préparation pharmacologique dans lequel l'agent réducteur de cholestérol est une solution liposomique d'un stérol ou d'un stanol associée à un agent émulsifiant et dissout, par homogénéisation dans du lait pour former des liposomes contenant l'agent réducteur de cholestérol.

12. Un produit comestible est préparé selon la revendication 11, **caractérisé en ce que** l'agent réducteur de cholestérol est une émulsion formée par un ou plusieurs stérols et/ou stanols, préférablement des stérols végétaux et /ou des stanols provenant du soja, colza et/d'huile de pins qui ont été émulsifiés avec un agent émulsifiant alimentaire comme des monoglycérides et de leurs dérivés avec des acides organiques, des esters de polyglycérol ou/et de la lécithine d'oeuf, de soja, de colza dans un rapport émulsifiant stérol/stanol qui est plus grand que 1:1, préférablement 2:1.

13. Un produit comestible selon les revendications 12 ou 13, **caractérisé en ce que** l'émulsion réductrice de cholestérol sous forme d'une solution liposomique contenant un stérol/stanol est incluse avec une concentration en poids entre 0.1 et 10% ou préférablement entre 1 et 3% du produit utilisé comme additif de produits laitiers, de matières grasses de cuisson, de yaourts, de produits laitiers acidifiés, de lait en poudre, de crème en poudre, de boissons de chocolat en poudre, de bouillons, de produits d'aliments naturels, de préparations pharmacologiques.
